# EUROPÄISCHE PATENTSCHRIFT

(11) **EP 3 003 241 B1**
(45) Veröffentlichungstag und Bekanntmachung des Hinweises auf die Patenterteilung: **08.08.2018**
(21) Anmeldenummer: 14734404.8
(22) Anmeldetag: 06.06.2014
(51) Int. Cl.: A61G 13/08, A61G 13/04, A61G 13/06, A61G 7/018

(54) **VORRICHTUNG UND VERFAHREN ZUM STEUERN EINES OPERATIONSTISCHS**
DEVICE AND METHOD FOR CONTROLLING A SURGICAL TABLE
DISPOSITIF ET PROCÉDÉ DE COMMANDE D'UNE TABLE D'OPÉRATION

(30) Priorität: 06.06.2013 DE 102013105869
(43) Veröffentlichungstag der Anmeldung: 13.04.2016
(73) Patentinhaber: Maquet GmbH, 76437 Rastatt (DE)
(72) Erfinder: RUCH, Jürgen, 77652 Offenburg (DE); JÖRGER, Matthias, 77855 Achern (DE); WELSCH, Michael, 76571 Gaggenau (DE)
(74) Vertreter: Zacco GmbH
(86) Internationale Anmeldenummer: PCT/EP2014/061817
(87) Internationale Veröffentlichungsnummer: WO 2014/195455

(56) Entgegenhaltungen:
- EP-A2- 2 508 160
- US-A- 6 000 076
- US-B2- 7 089 612
- TrumpfMedical: "Trumpf Medical TruSystem 7000", Youtube, 28 May 2013 (2013-05-28), XP054977208, Retrieved from the Internet: URL:https://www.youtube.com/watch?v=2pFt_d kOq3A [retrieved on 2017-03-13]

## Beschreibung

Die Erfindung betrifft eine Vorrichtung und ein Verfahren zum Steuern eines Operationstischs, der mindestens drei mithilfe von Bedienelementen in ihrer Stellung veränderbare Komponenten und mindestens zwei Antriebseinheiten hat, mit deren Hilfe die Stellung der ersten Komponente und zweiten Komponente relativ zueinander und relativ zur dritten Komponente veränderbar ist. Der Operationstisch hat ferner eine Steuereinheit zum Steuern der Antriebseinheiten, wobei in der Steuereinheit mindestens eine erste Stellung der ersten Komponente und zweiten Komponente relativ zueinander und relativ zur dritten Komponente gespeichert ist.

Aus dem Dokument DE 199 55 116 A1 ist eine Steuereinheit zur Ansteuerung der Antriebe einer von einer Operationstischsäule abnehmbaren Patientenlagerfläche mit elektromotorisch verstellbaren Komponenten, bestehend aus einer Energieversorgung, einer Steuerung und einem Bediengerät, bekannt. Das Bediengerät ist in einen Transportwagen zum Transport der Operationstischlagerfläche integriert.

Aus dem Dokument DE 10 2007 062 200 A1 ist ein Operationstisch mit einer Vielzahl von mithilfe von Bedienelementen verstellbaren Komponenten bekannt. Der Zustand und/oder die Zustandsänderung mindestens eines Teils der Bedienelemente wird durch Sensoren erfasst, wobei die von den Sensoren erzeugten Signale einer Verarbeitungseinrichtung zugeführt werden.

Aus dem Dokument DE 10 2005 054 230 A1 sind ein Verfahren und eine Einrichtung zur bidirektionalen Infrarotdatenübertragung zwischen einem Operationstisch und einem Bediengerät bekannt. Der Operationstisch und das Bediengerät sind jeweils Teilnehmer einer Infrarotdatenübertragung und umfassen jeweils einen Infrarotsender und einen Infrarotempfänger.

Aus dem Dokument DE 10 2005 054 223 A1 ist eine Einrichtung zum Verstellen eines Operationstischs bekannt, der eine Operationstischsäule hat, auf der eine verstellbare Patientenlagerfläche lösbar angeordnet ist. Die Einrichtung umfasst ein Bediengerät zum Eingeben von Verstellbefehlen zum Verstellen von Komponenten des Operationstischs. Die Verstellbefehle können vom Bediengerät direkt zur verstellbaren Lagerfläche übertragen werden.

Aus dem Dokument DE 10 2005 053 754 A1 ist eine Einrichtung zum Verstellen der Patientenlagerfläche eines Operationstischs bekannt, die mehrere relativ zueinander verstellbare Segmente umfasst. Zumindest ein Teil der verstellbaren Segmente ist mit Aktuatoren verbunden, die zum Verstellen der zugehörigen Segmente ansteuerbar sind. Die Eingabevorrichtung hat Mittel zum Eingeben von körperteilbezogenen Verstellbefehlen, die mit der Verstellung der Lage eines Körperteils oder Körperabschnitts eines auf der Patientenlagerfläche liegenden Patienten assoziiert sind.

Sowohl bei stationären Operationstischen als auch bei bewegbaren Operationstischen und mobilen Operationstischen können elektromotorisch verstellbare Komponenten vorgesehen sein, wie beispielsweise eine in ihrer Länge elektromotorisch veränderbare Operationstischsäule zur Veränderung der Höhe einer auf der Operationstischsäule angeordneten Patientenlagerfläche, ein um zwei orthogonale Achsen verstellbare Operationstischsäulenkopf zur Veränderung der Neigung bzw. der Kantung der mit dem Operationstischsäulenkopf verbundenen Patientenlagerfläche und/oder elektromotorisch verstellbare Komponenten der Patientenlagerfläche.

Jedoch ist es bei einigen Operationen wünschenswert, einen Patienten durch eine entsprechende Verstellung der Komponenten eines Operationstischs wiederholt in eine Lage zu bringen, die der Patient bereits zuvor eingenommen hatte oder die bei vorhergehenden Operationen von anderen Patienten eingenommen worden sind, insbesondere dann, wenn diese Position relativ aufwendig mithilfe eines oder mehrerer Bedienelemente eingestellt worden ist. Ferner soll dabei sichergestellt werden, dass der Patient bei der Verstellung der Komponenten nicht gefährdet wird

Aufgabe der Erfindung ist es, eine Vorrichtung und Verfahren zum Steuern eines Operationstischs anzugeben, durch die ein einfaches und sicheres Verfahren von Komponenten des Operationstischs in zumindest eine erste Stellung dieser Komponenten möglich ist.

Diese Aufgabe wird durch eine Vorrichtung zum Steuern eines Operationstischs mit den Merkmalen den Anspruchs 1 und durch ein Verfahren zum Steuern eines Operationstischs mit den Merkmalen des unabhängigen Verfahrensanspruchs gelöst. Vorteilhafte Weiterbildungen sind in den abhängigen Ansprüchen angegeben.

Bei der Vorrichtung und dem Verfahren zum Steuern eines Operationstischs wird insbesondere mindestens eine erste Stellung zumindest der erste Komponente und der zweiten Komponente zueinander und zur dritten Komponente gespeichert. Diese Stellung ist vorzugsweise als Information über die Lage der Komponenten zueinander und/oder der Stellung der Antriebseinheiten, gespeichert.

Bei der Aktivierung eines ersten Bedienelements werden die Antriebseinheiten derart gesteuert, dass die Komponenten in die gespeicherte Stellung bewegt werden. Es ist mindestens ein Steuerparameter voreingestellt gespeichert, wobei die Antriebseinheiten zum Bewegen der Komponenten zumindest von einer von der gespeicherten ersten Stellung verschiedenen zweiten Stellung in die gespeicherte erste Stellung abhängig von dem gespeicherten Steuerparameter gesteuert werden. Dadurch kann sichergestellt werden, dass bei der Bewegung der Komponenten von der zweiten Stellung in die erste Stellung durch den Steuerparameter definierte Bedingungen eingehalten werden, so dass eine Gefährdung eines auf der durch zumindest ein Teil der Komponenten gebildeten Patientenlagerfläche liegenden Patienten zumindest dan vermieden wird, wenn die Komponenten von der zweiten Stellung in die erste Stellung bewegt werden.

Es ist besonders vorteilhaft, wenn der Steuerparameter mindestens eine bei der Bewegung der Komponenten einzuhaltende Bedingung umfasst. Dadurch kann eine solche Bedingung einfach festgelegt und bei einer Bewegung der Komponenten von der zweiten Stellung in die erste Stellung oder umgekehrt eingehalten werden, wodurch die Gefährdung eines auf einer zumindest einen Teil der Komponenten gebildeten Patientenlagerfläche liegenden Patienten vermieden werden kann.

Besonders vorteilhaft ist es, wenn die Steuerparameter zulässige Freiheitsgrade der Bewegung der Komponente, zulässige Winkelbereiche von Lagewinkeln der Komponenten zueinander, die Möglichkeit des zeitgleichen Bewegens der Komponenten, die Möglichkeit des seriellen Bewegens der Komponenten, das alternierende Bewegen der Komponenten, das sequenzielle Bewegen der Komponenten, zulässige Verstellgeschwindigkeiten der Komponenten, zulässige Verstellgeschwindigkeitsverläufe der Verstellgeschwindigkeiten der Komponenten, zulässige Neigungen der Komponenten im Raum und/oder gespeicherte Bewegungsablaufsequenzen zwischen mehreren Stellungen als Bedienungen umfasst, wobei die Bedingungen auch für einzelne Abschnitte des Gesamtbewegungsablaufs zwischen zwei Stellungen separat festgelegt werden können. Als Steuerparameter kann auch eine die Bewegung einzelner Komponenten, insbesondere die Bewegungsgeschwindigkeit der Bewegung der Komponente, begrenzen und kann als Bewegungsparameter bezeichnet werden. Insbesondere kann der Steuerparameter für verschiedene Verstellpositionen der Komponenten unterschiedliche definiert sein, insbesondere unterschiedliche Begrenzungsparameter oder eine andere Art der Ansteuerung der Antriebseinheiten ermöglichen. Dadurch ist eine sehr individuelle Anpassung des Steuerparameters für verschiedene Bewegungsabschnitte der Verstellbewegung der Komponenten auf einfache Art möglich.

Die erste und/oder zweite gespeicherte Stellung kann insbesondere eine Nullstellung des Operationstischs, eine Übergabestellung zur Übergabe einer Patientenlagerfläche des Operationstischs an einen Transportwagen, eine operationsspezifische Ausgangsstellung der Komponenten des Operationstischs und/oder eine operationsspezifische Eingriffsstellung sein. Diese Stellungen sind vorzugsweise mithilfe von Bedienelementen zum Bedienen des Operationstischs, insbesondere mithilfe einer Fernbedienung, vorzugsweise mithilfe einer drahtlosen Fernbedienung, auswählbar, wobei die Stellungen zumindest zum Teil als feste, unveränderbare Stellungen gespeichert oder als durch einen Benutzer definierbare Stellungen abspeicherbar sind. Dadurch ist eine sehr flexible Handhabung und einfache Bedienung des Operationstischs für verschiedene Operationen einfach möglich.

Besonders vorteilhaft ist es, wenn eine erste Stellung und mindestens eine zweite Stellung voreingestellt gespeichert sind, wobei die Steuereinheit die erste Stellung und die zweite Stellung abweichend durch mehrmaliges Betätigen des ersten Bedienelements einstellt. Alternativ kann die Steuereinheit die erste Stellung beim Betätigen des ersten Bedienelements und die zweite Stellung beim Betätigen des zweiten Bedienelements mithilfe der Antriebseinheiten einstellen. Dadurch ist eine sehr einfache und intuitive Bedienung des Operationstischs möglich, so dass ein besonders einfacher Wechsel zwischen der ersten Stellung und der zweiten Stellung bzw. gespeicherten weiteren Stellungen möglich ist.

Ferner ist es vorteilhaft, wenn die Steuereinheit beim Deaktivieren des ersten Bedienelements und/oder beim Deaktivieren des zweiten Bedienelements die Bewegung der Komponenten auch dann stoppt, wenn die erste gespeicherte Stellung und/oder die zweite Stellung der Komponenten noch nicht erreicht ist. Dadurch ist sichergestellt, dass die Verstellbewegung nur dann erfolgt, wenn das Bedienelement aktuell betätigt ist, so dass eine Gefährdung der Patienten durch unkontrolliertes oder unbeaufsichtigtes Verfahren der Komponenten vermieden wird.

Alternativ oder zusätzlich kann der Bewegungsablauf unterbrochen werden, wenn mindestens eine Komponente in eine Lage bewegt worden ist, die ihrer Lage der gespeicherten Stellung entspricht. Die Bewegung der weiteren Komponenten in eine Lage, die ihrer Lage der gespeicherten Stellung entspricht, erfolgt erst nach einem erneuten Betätigen des Bedienelements, wobei die Betätigung des der Stellung zugeordneten Bedienelements zumindest kurzzeitig unterbrochen sein muss.

Besonders vorteilhaft ist es, wenn eine beliebige mithilfe von weiteren oder von den weiteren Bedienelementen durch eine Bedienperson einstellbare Stellung der Komponenten als voreingestellte Stellung speicherbar ist. Dadurch kann eine Bedienperson, insbesondere eines mit der Operation betrauten Arztes, einfach eine für ihn für die Operation günstige Stellung der Komponenten abspeichern, die er nachfolgend wieder durch das Betätigen des zugeordneten Bedienelements auswählen kann, so dass die Steuereinheit die Antriebseinheiten dann so steuert, dass die Komponenten wieder in der gespeicherten Stellung angeordnet werden.

Ferner ist es vorteilhaft, wenn in der Steuereinheit eine von der ersten gespeicherten Stellung verschiedene zweite Stellung der ersten Komponente und der zweiten Komponente relativ zueinander und relativ zur dritten Komponente gespeichert ist, wenn eine Bedienperson mithilfe von weiteren Bedienelementen die Stellung der Komponenten von der ersten gespeicherten Stellung in die zweite gespeicherte Stellung ändert, wenn die Steuereinheit den dabei bewirkten Bewegungsablauf und/oder die dazu durch die Steuereinheit erzeugten Ansteuerinformationen zum Ansteuern der Antriebseinheiten speichert und wenn der Bewegungsablauf und/oder die Ansteuerinformation zum erneuten Ausführen der Bewegung zwischen den beiden Stellungen mithilfe des ersten Bedienelements und/oder zweiten Bedienelements abrufbar ist.

Dabei ist es besonders vorteilhaft, wenn der gespeicherte Bewegungsablauf bzw. die gespeicherte Ansteuerinformation der Bewegung der Komponenten von der ersten Stellung in die zweite Stellung für eine Bewegung der Komponenten von der zweiten Stellung in die erste Stellung invers ausgeführt wird. So ist eine einfache Möglichkeit der Konfiguration des Steuerparameters gegeben, indem der konkrete Bewegungsablauf für eine Bewegung zwischen den beiden Stellungen das Steuerparameter gespeichert wird. Die Bewegung zwischen der ersten Stellung und der zweiten Stellung zum Ermitteln des zu speichernden Bewegungsablaufs wird auch als sogenannten Lernfahrt bezeichnet, bei der die Bewegung bzw. die Ansteuerinformation der Antriebseinheiten aufgezeichnet wird, so dass diese für eine spätere Bewegung der Komponenten zwischen diesen beiden Stellungen in beiden Richtungen, d.h. von der ersten Stellung in die zweite Stellung und von der zweiten Stellung in die erste Stellung, genutzt werden kann. Dadurch ist eine besonders einfache und sichere Handhabung des Operationstischs möglich, wobei dem Operateur auf einfache Art und Weise der Komfort ermöglicht wird, gespeicherte Stellungen wiederholt sicher nach einem von ihm vorgegebenen Bewegungsablauf anzufahren.

Dabei ist es vorteilhaft, wenn bei einer Bewegung der Komponenten von der ersten gespeicherten Stellung in die zweite gespeicherte Stellung ein vollständig umgekehrter Bewegungsablauf der Bewegung der Komponenten erfolgt, wie bei einer Bewegung der Komponenten von der zweiten gespeicherten Stellung in die erste gespeicherte Stellung. Dadurch ist der bereits erwähnte inverse Bewegungsablauf zwischen den beiden Stellungen einfach realisierbar.

Ferner ist es vorteilhaft, wenn die erste Komponente und die zweite Komponente jeweils eine Komponente einer Patientenlagerfläche des Operationstischs sind und wenn die dritte Komponente eine Operationstischsäule und/oder ein Operationstischsäulenfuß ist. Die Komponenten der Patientenlagerfläche sind eine Kopfplatte, eine Rückenplatte, eine Mittelplatte, eine erste einteilige oder mehrteilige Beinplatte und/oder eine zweite einteilige oder mehrteilige Beinplatte. Die Patientenlagerfläche dient zur Lagerung eines Patienten und kann weitere oder alternative Komponenten umfassen. Besonders vorteilhaft ist es, wenn die erste Komponente eine mit dem Operationstischsäulenkopf der Operationstischsäule verbundene Mittelplatte ist und die zweite Komponente eine mit der Mittelplatte verbundene Rückenplatte ist. Alternativ oder zusätzlich kann die zweite Komponente eine mit der Mittelplatte verbundene einteilige oder mehrteilige Beinplatte sein.

Alternativ oder zusätzlich können zwischen den einzelnen Komponenten auch Verlängerungskomponenten zum Vergrößern des durch die jeweilige Komponente bereitgestellten Liegebereichs sein. Die dritte Komponente ist vorzugsweise eine Operationstischsäule, wobei zur Definition der Lage der Komponenten relativ zur Operationstischsäule ein Fußpunkt der Operationstischsäule oder ein Punkt im Operationstischsäulenkopf genutzt werden kann. Alternativ oder zusätzlich kann als dritte Komponente ein Operationstischsäulenfuß dienen, wobei als Operationstischsäulenfuß in diesem Zusammenhang auch eine Kontaktfläche angesehen wird, mit der die Operationstischsäule fest mit einer bauwerkseitigen Aufnahme zur Aufnahme der Operationstischsäule verbunden ist. Dadurch ist eine einfache Erfassung der Stellung der Komponenten relativ zueinander möglich, wodurch die Lage eines auf der Patientenlagerfläche liegenden Patienten einfach mithilfe der gespeicherten Stellungen vorgegeben werden kann.

Besonders vorteilhaft ist es, wenn der Operationstisch mindestens eine Positionserfassungseinheit hat. Die Positionserfassungseinheit erfasst die Stellung der Komponenten zueinander und/oder im Raum. Bei einer Erfassung der Stellung der Komponenten im Raum kann dies insbesondere durch eine Angabe der Position der Komponenten in einem Weltkoordinatensystem erfolgen. Die Positionserfassungseinheit erfasst vorzugsweise den Verstellweg, den zeitlichen Verlauf des Verstellwegs, die Verstellgeschwindigkeit und/oder den zeitlichen Verlauf der Verstellgeschwindigkeit eines als Antriebseinheit dienenden Motors, vorzugsweise eines als Antriebseinheit dienenden Linearantriebs, und speichert die erfasste Information. Die Positionserfassungseinheit hat mindestens einen Sensor, insbesondere einen Beschleunigungssensor, einen Lagesensor, einen Drehgeber und/oder einen Schrittzähler zum Zählen der Schritte eines Schrittmotors.

Besonders vorteilhaft ist es, wenn die Antriebseinheiten mindestens einen Schrittmotor umfassen und wenn die Steuereinheit beim Ändern der Steuerung der Komponenten die Schrittfolge und/oder den zeitlichen Verlauf der Schrittfolge des Schrittmotors erfasst und als Information speichert. Dadurch ist eine einfache Aufzeichnung des Bewegungsablaufs der durch den Schrittmotor angetriebenen Komponente möglich, so dass eine einfache Wiederholung dieses Bewegungsablaufs möglich ist. Die Steuereinheit erfasst zusätzlich oder alternativ die Zeitdauer der Aktivierung der jeweiligen Antriebseinheit und/oder die für diese Antriebseinheit aktivierte Geschwindigkeitsstufe und speichert dies als Information. Mithilfe dieser gespeicherten Information kann ein einmal ausgeführter Bewegungsablauf einfach reproduziert werden, wenn der gleiche Bewegungsablauf ausgeführt werden soll.

Das Verfahren mit den Merkmalen des unabhängigen Verfahrensanspruchs kann mit den zuvor für die Vorrichtung angegebenen vorteilhaften Weiterbildungen weitergebildet werden, wobei dieselben vorteilhaften Wirkungen erzielt werden.

Die Komponenten des Operationstischs sind mithilfe der Antriebseinheiten vorzugsweise elektromotorisch verstellbar. Die Komponenten umfassen insbesondere beispielsweise eine in ihrer Länge elektromotorisch veränderbare Operationstischsäule zur Veränderung der Höhe einer auf der Operationstischsäule angeordneten Patientenlagerfläche, einen um zwei orthogonale Achsen verstellbaren Operationstischsäulenkopf zur Veränderung der Neigung und der Kantung der mit dem Operationstischsäulenkopf verbundenen Patientenlagerfläche und/oder elektromotorisch verstellbare Komponenten der Patientenlagerfläche.

Die erste Komponente und die zweite Komponente können bei einer vorteilhaften Ausführungsform einer Erfindung gegenüber der dritten Komponente auch in einer Position angeordnet werden, in der sie in einer waagerechten Ebene um 180° gedreht worden sind und/oder an einer senkrechten Ebene gespiegelt angeordnet sind, sodass wenn die erste Komponente und die zweite Komponente Bestandteile der Patientenlagerfläche sind und die dritte Komponente eine Operationstischsäule ist, die Patientenlagerfläche um 180° gedreht auf der Operationstischsäule angeordnet ist. Anders gesagt, die Patientenlagerfläche kann in zwei unterschiedliche Ausrichtungen mit der Operationstischsäule, vorzugsweise mit dem Operationstischsäulenkopf, verbunden werden.

Beim Verbinden der Patientenlagerfläche mit der Operationstischsäule kann die Patientenlagerfläche mit Hilfe eines Transportwagens aus zwei unterschiedlichen Richtungen auf die Operationstischsäule aufgelegt und mit dieser verbunden werden. Die erste Ausrichtung wird dabei als Normal-Ausrichtung bezeichnet und die zweite Ausrichtung als Reverse-Ausrichtung.

Wird die Patientenlagerfläche um 180° gedreht mit der Operationstischsäule bezüglich der Anordnung der Lagerfläche zum Zeitpunkt des Speicherns einer Stellung der Komponenten mit der Operationstischsäule verbunden, so ist die Stellung der ersten und der zweiten Komponente relativ zur dritten Komponente an einer senkrechten Ebene, insbesondere bezüglich Kantung und Neigung der Komponenten bzw. der Patientenlagerfläche, zu spiegeln. Ebenso ist ein abgespeicherter Bewegungsablauf zwischen zwei gespeicherten Stellungen an dieser senkrechten Ebene zu spiegeln. Die senkrechte Ebene ist vorzugsweise so angeordnet, dass eine Querachse der Patientenlagerfläche und/oder eine Querachse der Mitteilplatte der Patientenlagerfläche in einer Nullstellung der Patientenlagerfläche in dieser senkrechten Ebene liegt. Diese Querachse ist vorzugsweise orthogonal zu derer Längsachse der Patientenlagerfläche bzw. der Mittelplatte. Somit können sowohl die gespeicherten Stellungen als auch die gespeicherten Bewegungsabläufe zwischen zwei Stellungen und der gespeicherte Steuerparameter, die in Normal-Ausrichtung der Patientenlagerfläche abgespeichert wurden, auch in Reverse-Ausrichtung der Patientenlagerfläche genutzt werden. Somit wird beim Abspeichern einer Stellung und/oder eines Bewegungsablaufs in Normal-Ausrichtung der Patientenlagerfläche in einer Reverse-Ausrichtung der Patientenlagerfläche entsprechend gespiegelt angefahren, d. h. der Bewegungsablauf der Bewegung der Komponenten relativ zueinander ist an der senkrechten Ebene gespiegelt. Ist z.B. ein Teil der Patientenlagerfläche durch andere und/oder zusätzliche Komponenten gebildet oder sind externe Vorrichtungen vorhanden, die die Bewegung der Komponenten des Operationstischs begrenzen, sodass eine gespeicherte Stellung der Komponenten nicht erreicht werden kann, ist vorzugsweise vorgesehen, dass eine Bewegung der Komponenten bis zu einer der gespeicherten Stellung der Komponenten am nächsten kommenden Stellung erfolgt. Vorzugsweise wird ein gespeicherter Bewegungsablauf soweit ausgeführt, bis eine Begrenzungsvorgabe der Bewegung mindestens einer Komponente erreicht ist. Dies ist insbesondere dann vorteilhaft, wenn ein geänderter Aufbau der Patientenlagerfläche oder externe Geräte die Bewegung der Komponenten einschränken. Die Komponenten werden dann in die der gespeicherten Stellung räumlich am nächsten kommende Stellung bewegt.

Weitere Merkmale und Vorteile der Erfindung ergeben sich aus der nachfolgenden Beschreibung, die die Erfindung anhand von Ausführungsbeispielen im Zusammenhang mit den beigefügten Figuren näher erläutert.

Es zeigen:
- Figur 1a: einen Operationstisch mit mehreren mithilfe von über eine drahtlose Fernbedienung bereitgestellten Bedienelementen verstellbaren Komponenten in einer Ausgangsposition;
- Figur 1b: die Fernbedienung und den Operationstisch nach Figur 1a nach einer Kippneigungsverstellung ausgehend von der Ausgangsposition nach Figur 1a;
- Figur 1c: die Fernbedienung und den Operationstisch nach den Figuren 1a und 1b in einer zweiten Verstellposition gegenüber der in Figur 1a gezeigten Ausgangsposition, wobei die Patientenlagerfläche um eine zu ihrer Längsachse orthogonal verlaufenden Drehachse zur Längsneigungsverstellung verschwenkt worden ist und zusätzlich Komponenten der Patientenlagerfläche um mehrere Drehachsen gegenüber der Mittelplatte der Patientenlagerfläche verschwenkt worden sind;
- Figur 2: eine schematische Seitenansicht des Operationstischs in einer Nullstellung;
- Figur 3: die schematische Seitenansicht des Operationstischs in einer Übernahmestellung;
- Figur 4: die schematische Seitenansicht des Operationstischs in einer Operationsausgangsstellung;
- Figur 5: eine schematische Seitenansicht des Operationstischs in einer ersten benutzerdefinierten Stellung;
- Figur 6: eine schematische Seitenansicht des Operationstischs in einer zweiten benutzerdefinierten Stellung;
- Figur 7: einen ersten Ablaufplan zum Steuern der Bewegung der Komponenten des Operationstischs ausgehend von einer nicht dargestellten Operationsstellung der Komponenten des Operationstischs in die in Figur 2 gezeigte Nullstellung des Operationstischs mit gleichzeitiger Bewegung der Neigung der Mittelplatte und der Rückenplatte;
- Figur 8: einen zweiten Ablaufplan zum Steuern der Bewegung der Komponenten des Operationstischs aus einer nicht dargestellten Operationstischstellung der Komponenten in die in Figur 2 dargestellte Nullstellung bei dem gegenüber dem Ablauf nach Figur 7 ein Zwischenstopp vorgesehen ist;
- Figur 9: einen Ablaufplan zum Steuern der Bewegungen der Komponenten des Operationstischs ausgehend von einer nicht dargestellten Operationsstellung der Komponenten in die Nullstellung nach Figur 2 mit Zwischenstopp und bei einer Aktivierung der Verstellantriebe der Komponenten nacheinander; und
- Figur 10: einen Ablaufplan zum Bewegen der Komponenten des Operationstischs 30 aus einer nicht dargestellten Operationsstellung in die in Figur 3 dargestellten Übernahmestellung.

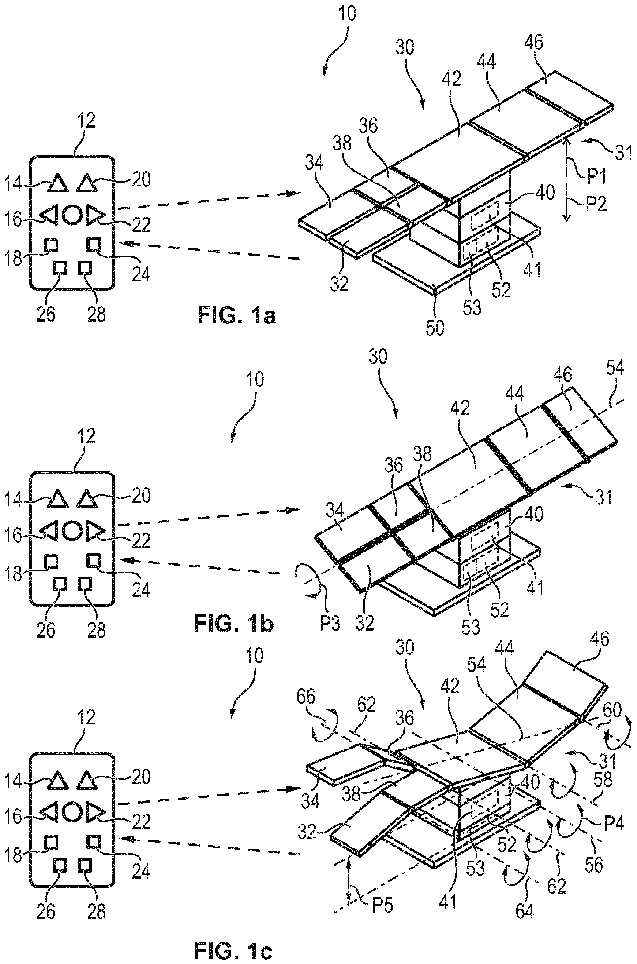

Figur 1a zeigt eine Anordnung 10 mit einer Fernbedienung 12, die mehrere Bedienelemente 14 bis 28 hat, mit deren Hilfe verstellbare Komponenten 32 bis 46 eines Operationstischs 30 verstellt, d.h. in ihrer Lage im Raum und/oder gegenüber anderen Komponenten 32 bis 46 geändert, werden können. Die einzelnen Komponenten 32 bis 46 oder Gruppen dieser Komponenten 32 bis 46 sind den Bedienelementen 14 bis 28 der Fernbedienung 12 zugeordnet, so dass beim Betätigen eines Bedienelements 14 bis 28 eine entsprechende Verstellaktion der diesem Bedienelement 14 bis 28 zugeordneten Komponente 32 bis 46 oder Komponentengruppe mithilfe einer dafür vorgesehenen Antriebseinheit ausgeführt wird. Beispielhaft ist die Antriebseinheit 41 zur Längenveränderung einer Operationstischsäule 40 dargestellt. Am unteren Ende der Operationstischsäule 40 ist ein Operationstischsäulenfuß 50 vorgesehen. Am gegenüberliegenden Ende ist die Operationstischsäule 40 mit einer die Komponenten 32 bis 36, 42 bis 46 umfassenden Patientenlagerfläche 31 verbunden. Mithilfe der Antriebseinheit 41 kann somit die Länge der Operationstischsäule 40 variiert und somit die Höhe der Patientenlagerfläche 31 über einem Fußboden, d. h. in Richtung der Pfeile P1 und P2, verändert werden, um einen auf der Patientenlagerfläche 31 liegenden Patienten in eine für eine durchzuführende Operation geeignete Position zu bringen.

Der Operationstisch 30 umfasst weitere nicht dargestellte Antriebseinheiten zum Verändern der Lage der Patientenlagerfläche 31, insbesondere zur Längsneigungsverstellung und/oder zur Kippneigungsverstellung der Patientenlagerfläche 31 sowie zur Verstellung von einzelnen Komponenten der Patientenlagerfläche 31 gegenüber weiteren Komponenten, wie dies insbesondere in Figur 1c dargestellt ist. In Figur 1b ist die Patientenlagerfläche 31 um ihre Längsachse 54 in Richtung des Pfeils P3 gedreht worden, so dass die Patientenlagerfläche 31 seitlich gekippt worden ist. Ein solch seitliches Kippen wird als Kippneigungsverstellung oder Kantung der Patientenlagerfläche 31 bezeichnet. Wie an der Mittelplatte 42 der Patientenlagerfläche 31 in Figur 1c zu erkennen ist, ist die Patientenlagerfläche 31 gegenüber Figur 1a um eine zur Längsachse 54 der Patientenlagerfläche 31 orthogonal verlaufenden Drehachse 56 in Richtung des Pfeils P4 geschwenkt worden, so dass eine Längsneigungsverstellung der Patientenlagerfläche 31 erfolgt ist. Diese Längsneigungsverstellung wird auch nur als Neigung der Patientenlagerfläche 31 bezeichnet. Ferner ist mithilfe der Antriebseinheit 41 die Länge der Operationstischsäule 40 verringert und somit die Patientenlagerfläche 31 in Richtung des Pfeils P2 abgesenkt worden.

Ferner ist die Lage des Rückenteils 44 gegenüber der Mittelplatte 42 durch eine Drehung um die Drehachse 58 und die Lage der Kopfplatte 46 gegenüber der Rückenplatte 44 der Patientenlagerfläche 31 durch eine Drehung um die Drehachse 60 verändert worden. Auch die Lage der die Segmente 34 und 36 bzw. 32 und 38 umfassenden Beinplatten ist gegenüber der Mittelplatte 42 der Patientenlagerfläche 31 durch eine entsprechende Drehung der Segmente 32 bis 38 um die Drehachsen 62, 64 und 66 geändert worden. Die verringerte Höhe der Patientenlagerfläche 31 ist durch den Pfeil P5 in Figur 1c angegeben.

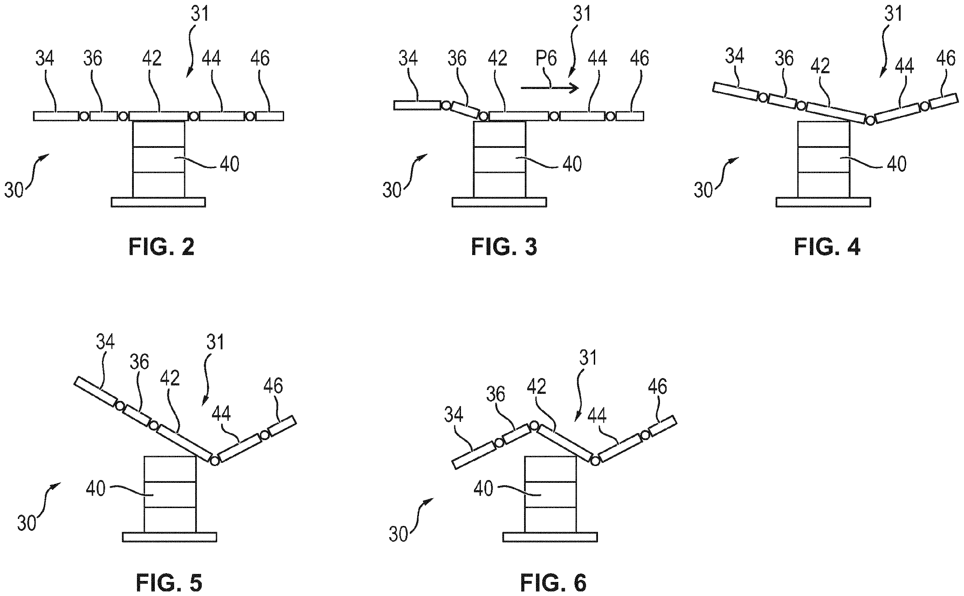

Figur 2 zeigt eine schematische Seitenansicht des Operationstischs 30, in der die Komponenten 32 bis 46 in einer sogenannten Nullstellung angeordnet sind, in der die Mittelplatte 42 mittig auf der Operationstischsäule 40 angeordnet ist.

Figur 3 zeigt eine schematische Seitenansicht des Operationstischs 30, in der die Komponenten 32 bis 46 in einer Übernahmestellung, angeordnet sind, in der die Komponenten 32 bis 38, 42 bis 46 der Patientenlagerfläche 31 durch ein Ausfahren der teleskopartigen Operationstischsäule 40 höher als in der Nullstellung angeordnet sind und wobei die Komponenten 32 bis 38, 42 bis 46 der Patientenlagerfläche 31 in Richtung des Pfeils P6, d. h. in Richtung der Längsachse 54 der Patientenlagerfläche 31, in eine Übernahmestellung zur Übernahme der Patientenlagerfläche durch einen Transportwagen zum Transport der von der Operationstischsäule 40 getrennten Patientenlagerfläche 31.

Figur 4 zeigt eine schematische Seitenansicht des Operationstischs 30, in der die Komponenten 32 bis 46 in einer Operationsausgangsstellung, angeordnet sind, in der die Patientenlagerfläche 31 um die Drehachse 56 gedreht worden ist, so dass die Mittelplatte 42 und die Beinplatten 32 bis 38 geneigt angeordnet sind und wobei das Rückenteil 44 mit dem Kopfteil 46 gegenüber der Mittelplatte 42 um die Drehachse 48 gedreht worden sind, so dass diese eine gegenüber der Mittelplatte 42 entgegengesetzte Neigung haben.

Figur 5 zeigt eine schematische Seitenansicht des Operationstischs 30, in der die Komponenten 32 bis 46 in einer ersten benutzerdefinierten Operationsstellung angeordnet sind, in der die Neigung der Mittelplatte 42 und der Beinplatten 32 bis 38 im Unterschied zur Operationsausgangsstellung nach Figur 4 vergrößert worden ist. Ferner ist auch die entgegengesetzte Neigung der Rückenplatte 44 mit der Kopfplatte 46 zu den Beinplatten 32 bis 38 vergrößert worden.

Figur 6 zeigt eine schematische Seitenansicht des Operationstischs 30, in der die Komponenten 32 bis 46 in einer zweiten benutzerdefinierten Operationsstellung angeordnet sind, in der die Beinplatten 32 bis 38 im Unterschied zur ersten benutzerdefinierten Stellung gegenüber der Mittelplatte 42 abgewinkelt sind, so dass sie die gleiche Neigung aufweisen wie die Rückenplatte 44 und die Kopfplatte 46.

Im vorliegenden Ausführungsbeispiel ist die in Figur 2 dargestellte Nullstellung dem Bedienelement 18, die in Figur 4 gezeigte Operationsausgangsstellung dem Bedienelement 26, die in Figur 5 gezeigte erste benutzerdefinierte Operationsstellung dem Bedienelement 28 und die in Figur 6 dargestellte zweite benutzerdefinierte Operationsstellung dem Bedienelement 24 der Fernbedienung 12 zugeordnet. Dadurch können die Nullstellung, die Operationsausgangsstellung, die erste benutzerdefinierte Stellung, sowie die zweite benutzerdefinierte Stellung einfach durch Aktivieren des entsprechenden Bedienelements 18, 24 bis 28 aus jeder beliebigen Stellung eingestellt werden. Die Operationsausgangsstellung, die erste benutzerdefinierte Operationsstellung und die zweite benutzerdefinierte Operationsstellung sind mithilfe der weiteren Bedienelemente 14, 16, 20, 22 der Fernbedienung einmalig eingestellt und dann dem jeweiligen Bedienelement 18, 24, 26, 28 zugeordnet gespeichert worden, so dass diese jeweilige Stellung der Komponenten 32 bis 46 mithilfe der Bedienelemente 18, 24, 26, 28 aktivierbar und somit auch während einer Operation einfach mithilfe der Fernbedienung 12 einstellbar ist. Dabei erfolgt die Bewegung aus einer beliebigen Stellung in die abgespeicherte Stellung der Komponenten 32 bis 46 aus jeder beliebigen Stellung nur solange, wie das jeweilige Bedienelement 18, 24, 26, 28 aktiviert ist. Die Bewegung wird somit sofort gestoppt, wenn das jeweilige Bedienelement 18, 24, 26, 28 nicht mehr betätigt wird. Bei alternativen Ausführungsformen kann die Bewegung solange fortgesetzt werden, bis eine Zwischenstellung oder die dem jeweiligen Bedienelement 18, 24, 26, 28 zugeordnete Endstellung, d.h. die Nullstellung, die Operationsausgangsstellung, die erste benutzerdefinierte Operationsstellung bzw. die zweite benutzerdefinierte Operationsstellung, erreicht worden ist.

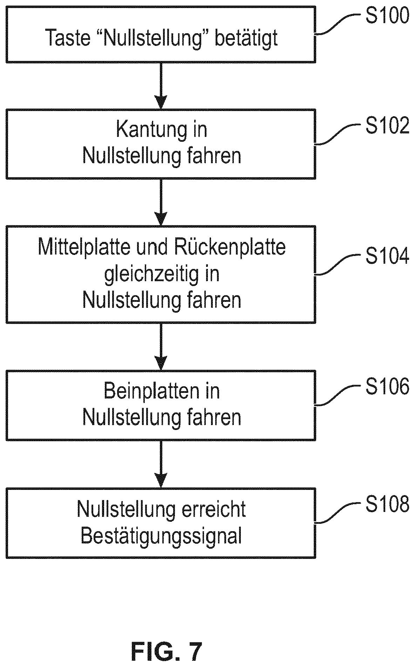

Figur 7 zeigt einen Ablaufplan zum Verfahren der Komponenten 32 bis 46 des Operationstischs 30 in die in Figur 2 gezeigte Nullstellung. Der Ablauf wird im Schritt S100 damit gestartet, dass das der Nullstellung zugeordnete Bedienelement 18 durch eine Bedienperson betätigt worden ist. Anschließend wird die Kantung des Operationstischs 30 in Schritt S102 in die Nullstellung bewegt, d.h. die Patientenlagerfläche 31 wird um die Drehachse 54 gedreht, bis die zu dieser orthogonalen Drehachse 56 in einer waagerechten Ebene angeordnet ist.

Anschließend wird gleichzeitig die Neigung der Patientenlagerfläche 31 bzw. der Mittelplatte 42 der Patientenlagerfläche 31 im Schritt S104 um die Drehachse 56 gedreht, bis auch die Längsachse 60 der Patientenlagerfläche 31 in einer waagerechten Ebene angeordnet ist. Gleichzeitig wird die Rückenplatte 44 um die Drehachse 58 in Nullstellung geändert, so dass anschließend sowohl die Oberflächen der Mittelplatte 42 als auch der Rückenplatte 44 waagerecht ausgerichtet sind.

Nachfolgend werden die Beinplatten 32 bis 38 mithilfe der entsprechenden Antriebe um die Achsen 62, 64, 66 gedreht, bis auch die Oberflächen dieser Beinplatten 32 bis 38 waagerecht angeordnet sind, so dass anschließend im Schritt S108 die Nullstellung des Operationstischs 30 erreicht worden ist und die Steuereinheit 52 des Operationstischs 30 ein Bestätigungssignal über die Fernbedienung 12 oder ein anderes geeignetes Ausgabemittel ausgibt.

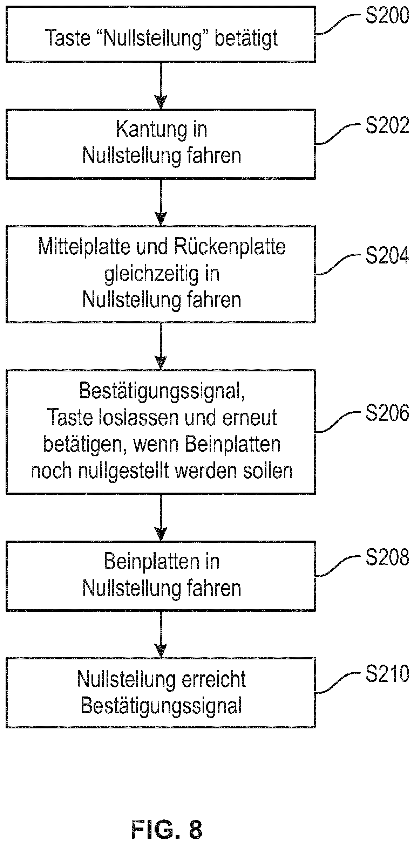

In Figur 8 ist ein Ablaufplan eines Ablaufs zum Bewegen der Komponenten 32 bis 46 des Operationstischs 30 in die Nullstellung alternativ zu dem in Figur 7 dargestellten Ablauf gezeigt. Die Schritte S200 bis S210 des Ablaufs nach Figur 8 unterscheiden sich von dem Ablauf nach Figur 7 durch den zusätzlichen Schritt S206. Die Schritte S200 bis S204 stimmen mit den Schritten S100 bis S104 und die Schritte S208 und S210 mit den Schritten S106 und S108 überein. Nach dem gleichzeitigen Verfahren der Mittelplatte 42 und der Rückenplatte 44 in die jeweiligen Nullstellungen im Schritt S204 wird im Schritt S206 ein Bestätigungssignal ausgegeben und keine weitere Bewegung der Komponenten 32 bis 46 in die Nullstellung aktiviert, bis das Bedienelement 18 erneut betätigt worden ist. Dazu muss das Betätigungselement 18 kurzzeitig nicht betätigt und erneut wieder betätigt werden, wenn auch die weiteren Komponenten, im vorliegenden Ausführungsbeispiel die Beinplatten 32 bis 38, nachfolgend in Nullstellung gebracht werden sollen. Durch das Stoppen der Bewegung nach der Bewegung der Mittelplatte 42 und der Rückenplatte 44 in Nullstellung kann der Zustand des Patienten nochmals kontrolliert werden, bevor die Bewegung der weiteren Komponenten 32 bis 38 in ihre Nullstellung aktiviert wird. Das nachfolgende Bewegen der Beinplatten 32 bis 38 in ihre Nullstellung in Schritt S208 und die nachfolgende Ausgabe des Bestätigungssignals im Schritt S210 erfolgt in gleicher Weise wie in Verbindung mit Figur 7 bereits beschrieben.

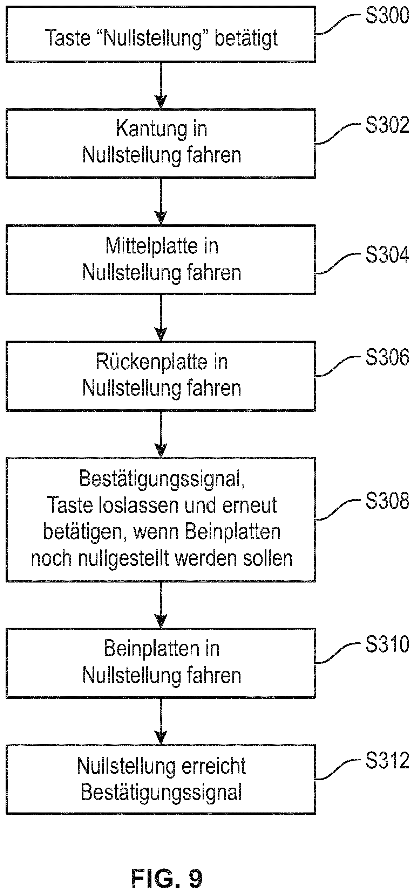

In Figur 9 ist ein Ablaufplan zum Ablauf der Bewegungssteuerung der Komponenten 32 bis 46 des Operationstischs 30 in die Nullstellung dargestellt. Die Schritte S300 und S302 stimmen mit den Schritten S200 und S202 und die Schritte S308 bis S312 mit den Schritten S206 bis S210 des Ablaufs nach Figur 8 überein. Die gleichzeitige Bewegung der Neigung der Mittelplatte 42 und der Rückenplatte 44 im Schritt S204 erfolgt beim Ablauf nach Figur 9 im Unterschied zum Ablauf nach Figur 8 nicht gleichzeitig sondern nacheinander, wobei zuerst im Schritt S304 die Mittelplatte in die Nullstellung bewegt und anschließend im Schritt S306 die Rückenplatte 44 in ihre Nullstellung bewegt wird.

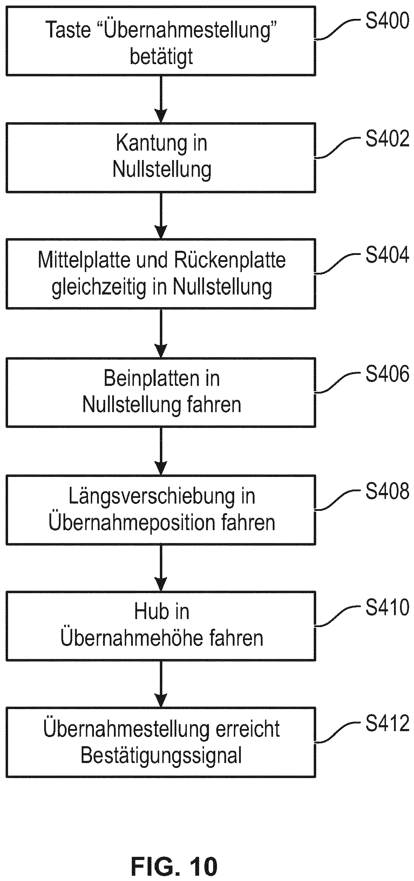

In Figur 10 ist ein Ablaufplan zur Steuerung der Bewegung der Komponenten 32 bis 46 in die in Figur 3 gezeigte Übernahmestellung gezeigt. Der Ablauf wird im Schritt S400 mit dem Aktivieren des der Übernahmestellung zugeordneten Bedienelements 26 der Fernbedienung 12 gestartet. Anschließend wird die Kantung der Mittelplatte 42 im Schritt S402 in Nullstellung gebracht. Anschließend werden im Schritt S404 die Neigung der Mittelplatte 42 und der Rückenplatte 44 gleichzeitig in Nullstellung gebracht. Nachfolgend werden im Schritt S406 die Beinplatten 32 bis 38 in Nullstellung bewegt. Anschließend erfolgt eine Längsverschiebung der Patientenlagerfläche 31 in Richtung des Pfeils P6 und nachfolgend eine Änderung der Höhe der Patientenlagerfläche 31 in die in der Figur 3 gezeigte gegenüber der Nullstellung höheren Übernahmeposition, indem mithilfe der Antriebseinheit 41 der Operationstischsäule 40 die Operationstischsäule 40 auf die entsprechende Höhe ausgefahren wird. Nach Erreichen der Übernahmestellung im Schritt S412 wird ein Bestätigungssignal durch den Operationstisch 30 und/oder die Fernbedienung 12 ausgegeben. In gleicher Weise wie in Verbindung mit den Figuren 8 und 9 beschrieben, kann auch für den Ablauf nach Figur 10 nach dem Erreichen einer Nullstellung einer Komponente 42, 44, die Bewegung der weiteren Komponenten 32 bis 38 gestoppt werden, bis ein erneutes Eingabesignal erfolgt. Eine solche Unterbrechung der Bewegung kann auch bei allen in den Figuren 7 bis 10 gezeigten Abläufen nach dem Erreichen einer Zwischenposition oder einer Endposition einer Komponente 32 bis 46 erfolgen, um den Bediener dazu anzuhalten, den Zustand des Patienten vor einer weiteren Bewegung der Komponenten 32 bis 46 des Operationstischs 30 zu kontrollieren.

Die erste Komponente 42 und die zweite Komponente 44 können bei einer vorteilhaften Ausführungsform einer Erfindung gegenüber der dritten Komponente 40 auch in einer Position angeordnet sein, in der sie in einer waagerechten Ebene um 180° gedreht worden sind und/oder an einer senkrechten Ebene gespiegelt angeordnet sind, sodass wenn die erste Komponente 42 und die zweite Komponente 44 Bestandteile der Patientenlagerfläche 31 des Operationstischs 30 sind und die dritte Komponente eine Operationstischsäule 40 ist, die Patientenlagerfläche 31 um 180° gedreht auf der Operationstischsäule 40 angeordnet ist. Anders gesagt, die Patientenlagerfläche 31 kann in zwei unterschiedliche Ausrichtungen mit der Operationstischsäule 40, vorzugsweise mit dem Operationstischsäulenkopf, verbunden werden.

Beim Verbinden der Patientenlagerfläche 31 mit der Operationstischsäule 30 kann die Patientenlagerfläche 31 mit Hilfe eines Transportwagens aus zwei unterschiedlichen Richtungen auf die Operationstischsäule 40 aufgesetzt und mit dieser verbunden werden. Die erste Ausrichtung wird dabei als Normal-Ausrichtung bezeichnet und die zweite Ausrichtung als Reverse-Ausrichtung.

Wird die Patientenlagerfläche 31 um 180° gedreht mit der Operationstischsäule 40 bezüglich der Anordnung der Patientenlagerfläche 31 zum Zeitpunkt des Speicherns einer Stellung der Komponenten 32 bis 46 mit der Operationstischsäule 40 verbunden, so ist die Stellung der ersten und der zweiten Komponente 42, 44 relativ zur dritten Komponente 40 an einer senkrechten Ebene, insbesondere bezüglich Kantung und Neigung der Komponenten 32 bis 38, 42 bis 46 bzw. der Patientenlagerfläche 31, zu spiegeln. Ebenso ist ein abgespeicherter Bewegungsablauf zwischen zwei gespeicherten Stellungen an dieser senkrechten Ebene zu spiegeln. Die senkrechte Ebene ist vorzugsweise so angeordnet, dass eine Querachse der 56 Patientenlagerfläche 31 und/oder eine Querachse 56 der Mitteilplatte 42 der Patientenlagerfläche 31 in einer Nullstellung der Patientenlagerfläche 31 in dieser senkrechten Ebene liegt. Diese Querachse 56 ist vorzugsweise orthogonal zu derer Längsachse 54 der Patientenlagerfläche bzw. der Mittelplatte 42. Somit kann sowohl die gespeicherten Stellungen als auch die gespeicherten Bewegungsabläufe zwischen zwei Stellungen und der gespeicherte Steuerparameter, die in Normal-Ausrichtung der Patientenlagerfläche 31 abgespeichert wurden, auch in Reverse-Ausrichtung der Patientenlagerfläche 31 genutzt werden. Somit wird beim Abspeichern einer Stellung und/oder eines Bewegungsablaufs in Normal-Ausrichtung der Patientenlagerfläche 31 in einer Reverse-Ausrichtung der Patientenlagerfläche 31 entsprechend gespiegelt angefahren, d. h. der Bewegungsablauf der Bewegung der Komponenten 32 bis 46 relativ zueinander ist an der senkrechten Ebene gespiegelt. Ist z.B. ein Teil der Patientenlagerfläche 31 durch andere und/oder zusätzliche Komponenten gebildet oder sind externe Vorrichtungen vorhanden, die die Bewegung der Komponenten 32 bis 46 des Operationstischs 30 begrenzen, sodass eine gespeicherte Stellung der Komponenten 32 bis 46 nicht erreicht werden kann, ist vorzugsweise vorgesehen, dass eine Bewegung der Komponenten 32 bis 46 bis zu einer der gespeicherten Stellung der Komponenten 32 bis 46 am nächsten kommenden Stellung erfolgt. Vorzugsweise wird ein gespeicherter Bewegungsablauf soweit ausgeführt, bis eine Begrenzungsvorgabe der Bewegung mindestens einer Komponente 32 bis 46 erreicht ist. Dies ist insbesondere dann vorteilhaft, wenn ein geänderter Aufbau der Patientenlagerfläche 31 oder externe Geräte die Bewegung der Komponenten 32 bis 46 einschränken. Die Komponenten 32 bis 46 werden dann in die der gespeicherten Stellung räumlich am nächsten kommende Stellung bewegt.

### Bezugszeichenliste

- 10: Operationstisch
- 12: Fernbedienung
- 14 bis 28: Bedienelement
- 30: Operationstisch
- 31: Patientenlagerfläche
- 32 bis 38, 42 bis 46: Komponenten
- 40: Operationstischsäule
- 41: Antriebseinheit
- 50: Operationstischsäulenfuß
- 52: Steuereinheit
- 53: Akkumulator
- 54: Längsachse der Patientenlagerfläche
- 56: Querachse der Patientenlagerfläche
- 58 bis 66: Schwenkachsen
- P1 bis P6: Richtungspfeile
- S100 bis S412: Verfahrensschritte

## Patentansprüche

1. Vorrichtung zum Steuern eines Operationstischs, umfassend einen Operationstisch (30) mit mindestens drei mithilfe von Bedienelementen (14 bis 28) in ihrer Stellung veränderbare Komponenten (32 bis 46), mindestens zwei Antriebseinheiten (41), mit deren Hilfe die Stellung der ersten Komponente (42) und zweiten Komponente (44) relativ zueinander und relativ zur dritten Komponente (40) veränderbar ist,
eine Steuereinheit (52) zum Steuern der Antriebseinheiten (41), wobei in der Steuereinheit (52) mindestens eine erste Stellung der ersten Komponente (42) und zweiten Komponente (44) relativ zueinander und relativ zur dritten Komponente (40) gespeichert ist, wobei die Steuereinheit (52) bei der Aktivierung eines ersten Bedienelements (18) die Antriebseinheiten (41) derart steuert, dass die Komponenten (32 bis 46) in die gespeicherte Stellung bewegt werden,
wobei in der Steuereinheit (52) mindestens ein Steuerparameter voreingestellt gespeichert ist, und wobei die Steuereinheit (52) die mindestens zwei Antriebseinheiten (41) zum Bewegen der Komponenten (32 bis 46) zumindest von einer von der gespeicherten ersten Stellung verschiedenen zweiten Stellung in die gespeicherte erste Stellung abhängig von dem gespeicherten Steuerparameter steuert, **dadurch gekennzeichnet, dass** die Antriebseinheiten (41) mindestens einen Schrittmotor umfassen und die Steuereinheit (52) beim Ändern der Stellung der Komponenten (32 bis 46) die Schrittfolge und/oder den zeitlichen Verlauf der Schrittfolge des Schrittmotors erfasst und als Information speichert, und/oder
wobei die Steuereinheit (52) beim Ausführen jeder Lageänderung der Komponenten (32 bis 46) den Verstellweg, den zeitlichen Verlauf des Verstellwegs, die Verstellgeschwindigkeit und/oder den zeitlichen Verlauf der Verstellgeschwindigkeit eines als Antriebseinheit (41) dienenden Linearantriebs, insbesondere Hydraulikzylinders oder Spindelantriebs, erfasst und als Information speichert, und
wobei die Steuereinheit (52) zusätzlich oder alternativ die Zeitdauer der Aktivierung der jeweiligen Antriebseinheit (41) und/oder die für diese Antriebseinheit (41) aktivierte Geschwindigkeitsstufe erfasst und als Information speichert.

2. Vorrichtung nach Anspruch 1, **dadurch gekennzeichnet, dass** der Steuerparameter mindestens eine bei der Bewegung der Komponenten (32 bis 46) einzuhaltende Bedingung umfasst.

3. Vorrichtung nach Anspruch 1 oder 2, **dadurch gekennzeichnet, dass** der Steuerparameter wenigstens eine aus den folgenden Optionen umfasst:
zulässige Freiheitsgrade der Bewegung einer Komponente (32 bis 46),
zulässige Winkelbereiche von Lagewinkeln der Komponenten (32 bis 46) zueinander,
das parallele Bewegen der Komponenten (32 bis 46),
das serielle Bewegen der Komponenten (32 bis 46),
alternierende Bewegen der Komponenten (32 bis 46),
das sequentielle Bewegen der Komponenten (32 bis 46),
zulässige Verstellgeschwindigkeiten der Komponenten (32 bis 46),
zulässige Verstellgeschwindigkeitsverläufe der Verstellgeschwindigkeit der Komponenten (32 bis 46),
zulässige Neigungen der Komponenten (32 bis 46) im Raum, und/oder
gespeicherte Bewegungsablaufsequenzen zwischen mehreren Stellungen der Komponenten (32 bis 46).

4. Vorrichtung nach einem der vorhergehenden Ansprüche, **dadurch gekennzeichnet, dass** als Stellung eine Nullstellung des Operationstischs (30), eine Übergabestellung zur Übergabe einer Patientenlagerfläche (31) des Operationstischs (30) an einen Transportwagen, eine operationsspezifische Ausgangsstellung des Operationstischs (30) und/oder eine operationsspezifische Eingriffsstellung des Operationstischs (30) gespeichert ist.

5. Vorrichtung nach einem der vorhergehenden Ansprüche, **dadurch gekennzeichnet, dass** eine erste Stellung und mindestens eine zweite Stellung voreingestellt gespeichert sind, wobei die Steuereinheit (52) die erste Stellung und die zweite Stellung nacheinander durch mehrmaliges Betätigen des ersten Bedienelements (18) oder wobei die Steuereinheit (52) die erste Stellung beim Betätigen des ersten Bedienelements (18) und die zweite Stellung beim Betätigen des zweiten Bedienelements (24) mithilfe der Antriebseinheiten (41) einstellt.

6. Vorrichtung nach einem der vorhergehenden Ansprüche, **dadurch gekennzeichnet, dass** die Steuereinheit (52) beim Deaktivieren des ersten und/oder zweiten Bedienelements (18, 24) die Bewegung der Komponenten (32 bis 46) auch dann stoppt, wenn die erste gespeicherte Stellung und/oder die zweite Stellung der Komponenten (32 bis 46) noch nicht erreicht ist.

7. Vorrichtung nach einem der vorhergehenden Ansprüche, **dadurch gekennzeichnet, dass** eine beliebige mithilfe von weiteren oder von den weiteren Bedienelementen (14 bis 22) durch eine Bedienperson einstellbare Stellung der Komponenten (32 bis 46) als voreingestellte Stellung speicherbar ist.

8. Vorrichtung nach einem der vorhergehenden Ansprüche, **dadurch gekennzeichnet, dass** in der Steuereinheit (52) eine von der ersten gespeicherten Stellung verschiedene zweite Stellung der ersten Komponente (42) und der zweiten Komponente (44) relativ zueinander und relativ zur dritten Komponente (40) gespeichert ist, dass eine Bedienperson mithilfe von weiteren Bedienelementen (14 bis 22) die Stellung der Komponenten (40, 42, 44) von der ersten gespeicherten Stellung in die zweite gespeicherte Stellung ändert, dass die Steuereinheit (52) den dabei erzeugten Bewegungsablauf und/oder die dazu durch die Steuereinheit (52) erzeugte Ansteuerinformation zum Ansteuern der Antriebseinheiten (41) speichert, und dass der Bewegungsablauf und/oder die Ansteuerinformation zum erneuten Ausführen der Bewegung zwischen den beiden Stellungen mithilfe des ersten Bedienelements (18) und/oder zweiten Bedienelements (24) abrufbar ist.

9. Vorrichtung nach Anspruch 8, **dadurch gekennzeichnet, dass** bei einer Bewegung der Komponenten (32 bis 46) von der ersten gespeicherten Stellung in die zweite gespeicherte Stellung ein vollständig umgekehrter Bewegungsablauf der Bewegung der Komponenten (32 bis 46) erfolgt wie bei der Bewegung der Komponenten (32 bis 46) von der zweiten gespeicherten Stellung in die erste gespeicherte Stellung.

10. Vorrichtung nach einem der vorhergehenden Ansprüche, **dadurch gekennzeichnet, dass** die erste Komponente (42) und die zweite Komponente (44) jeweils eine Komponente einer Patientenlagerfläche (31) des Operationstischs (30) sind, und dass die dritte Komponente eine Operationstischsäule (40) und/oder ein Operationstischsäulenfuß (50) ist.

11. Vorrichtung nach einem der vorhergehenden Ansprüche, **dadurch gekennzeichnet, dass** der Operationstisch (30) mindestens eine Positionserfassungseinheit, die die Stellung der Komponenten (32 bis 46) zueinander und/oder im Raum erfasst, hat, wobei die Positionserfassungseinheit mindestens einen Sensor umfasst.

12. Verfahren zum Steuern eines Operationstischs, der mindestens drei mithilfe von Bedienelementen (14 bis 28) in ihrer Lage veränderbare Komponenten (32 bis 46) hat, bei dem mithilfe mindestens zweier Antriebseinheiten (41) die Stellung der ersten Komponente und der zweiten Komponente (44) relativ zueinander und relativ zur dritten Komponente (40) verändert werden kann, die Antriebseinheiten (41) mithilfe einer Steuereinheit (52) gesteuert werden, mindestens eine erste Stellung zumindest der ersten Komponente (42) und der zweiten Komponente (44) relativ zueinander und relativ zur dritten Komponente (40) in der Steuereinheit (52) gespeichert wird, die mindestens zwei Antriebseinheiten (41) beim Aktivieren eines ersten Bedienelements (18) durch die Steuereinheit derart gesteuert werden, dass die Komponenten (32 bis 46) in die gespeicherte Stellung bewegt werden, in der Steuereinheit (52) mindestens ein Steuerparameter voreingestellt gespeichert wird, und bei dem die mindestens zwei Antriebseinheiten (41) zum Bewegen der Komponenten (32 bis 46) zumindest von einer von der gespeicherten ersten Stellung verschiedenen zweiten Stellung in die gespeicherte erste Stellung durch die Steuereinheit (52) abhängig von dem gespeicherten Steuerparameter gesteuert werden,
wobei die Antriebseinheiten (41) mindestens einen Schrittmotor umfassen und die Steuereinheit (52) beim Ändern der Stellung der Komponenten (32 bis 46) die Schrittfolge und/oder den zeitliche Verlauf der Schrittfolge des Schrittmotors erfasst und als Information speichert, und/oder
wobei die Steuereinheit (52) beim Ausführen jeder Lageänderung der Komponenten (32 bis 46) den Verstellweg, den zeitlichen Verlauf des Verstellwegs, die Verstellgeschwindigkeit und/oder den zeitlichen Verlauf der Verstellgeschwindigkeit eines als Antriebseinheit (41) dienenden Linearantriebs, insbesondere Hydraulikzylinders oder Spindelantriebs, erfasst und als Information speichert, und
wobei die Steuereinheit (52) zusätzlich oder alternativ die Zeitdauer der Aktivierung der jeweiligen Antriebseinheit (41) und/oder die für diese Antriebseinheit (41) aktivierte Geschwindigkeitsstufe erfasst und als Information speichert.

## Claims

1. Device for controlling a surgical table,
comprising a surgical table (30) having
at least three components (32 through 46), the position of which can be modified with the assistance of operating elements (14 through 28),
at least two drive units (41), with the assistance of which the position of the first component (42) and the second component (44) can be modified relative to one another and relative to the third component (40),
a control unit (52) for controlling the drive units (41), whereby in the control unit (52) at least a first position of the first component (42) and second component (44) relative to one another and relative to the third component (40) is stored, whereby
upon actuation of a first operating element (18), the control unit (52) controls the drive units (41) in such a way that the components (32 through 46) are moved into the stored position, whereby at least one preset control parameter is stored in the control unit (52), and whereby the control unit (52) controls the at least two drive units (41) to move the components (32 through 46) at least from one of the second positions that are different from the stored first position, into the first stored position, depending on the stored control parameter, **characterized by** the fact that
the drive units (41) comprise at least one stepper motor and upon modification of the position of the components (32 through 46), the control unit (52) records and stores, as information, the sequence of steps and/or the timing of the sequence of steps of the stepper motor and/or
whereby, upon execution of every positional modification of the components (32 through 46), the control unit (52) records and saves as information the adjustment travel, the timing of the adjustment travel, the adjustment travel velocity and/or the timing of the adjustment travel velocity of a linear drive serving as a drive unit (41), in particular hydraulic cylinders or screw drives, and
whereby the control unit (52) additionally or alternatively records and saves as information, the duration of the actuation of the respective drive unit (41) and/or velocity level actuated for this drive unit (41).

2. Device according to Claim 1, **characterized by** the fact that the control parameter comprises at least one of the specifications that must be observed in the movement of the components (32 through 46).

3. Device according to Claim 1 or 2, **characterized by** the fact that the control parameter comprises at least one of the following options:
acceptable degree of freedom of motion of a component (32 through 46),
acceptable angular range of the orientation angles of the components (32 through 46) relative to one another,
the parallel movement of the components (32 through 46),
the serial movement of the components (32 through 46),
alternating movement of the components (32 through 46),
the sequential movement of the components (32 through 46),
acceptable travel velocities of the components (32 through 46),
acceptable courses of the travel velocity of the travel velocity of the components (32 through 46),
acceptable inclinations of the components (32 through 46) in space and/or
stored movement sequence runs between multiple positions of the components (32 through 46).

4. Device according to one of the preceding Claims, **characterized by** the fact that the following positions are stored: zero position of the surgical table (30), a transfer position for the transfer of a patient seating surface (31) of the surgical table (30) to a transport cart, an initial surgery-specific initial position of the surgical table (30) and/or a surgery-specific intervention position of the surgical table (30).

5. Device according to one of the preceding Claims, **characterized by** the fact that a preset first position and at least a second preset position are stored, whereby the control unit (52) engages the first position and the second position successively by means of repeated activation of the first operating element (18) or whereby the control unit (52) engages the first position upon activation of the first operating element (18) and the second position upon activation of the second operating element (24) with the assistance of the drive units (41).

6. Device according to one of the preceding Claims, **characterized by** the fact that upon deactivation of the first and/or second operating element (18, 24), the control unit (52) then also stops the movement of the components (32 through 46), when the first stored position and/or the second position of the components (32 through 46) have/has not yet been reached.

7. Device according to one of the preceding Claims, **characterized by** the fact that a random position of the components (32 through 46) that can be adjusted by an operator, can be stored as a preset position with the assistance from further, or from the further operating element(s) (14 through 22) .

8. Device according to one of the preceding Claims, **characterized by** the fact that a second position, that differs from the first stored position, of the first component (42) and of the second component (44), relative to one another and relative to the third component (40), is stored in the control unit (52), that an operator with the assistance of further operating elements (14 through 22) modifies the position of the components (40, 42, 44) from the first stored position to the second stored position, that the control unit (52) stores the thereby generated movement sequence and/or the control information for the control of the drive units (41), produced by the control unit (52), and that the movement sequence and/or the control information for the new execution of the movement between the two positions with assistance of the first operating element (18) and/or second operating element (24) is retrievable.

9. Device according to Claim 8, **characterized by** the fact that upon a movement of the components (32 through 46) from the first stored position into the second stored position, an entirely opposite movement sequence of the components (32 through 46) occurs, as in the case of the movement of the components (32 through 46) from the second stored position into the first stored position.

10. Device according to one of the preceding Claims, **characterized by** the fact that the first component (42) and the second component (44) are respectively a component of a patient seating surface (31) of the surgical table (30), and that the third component is a surgical table pedestal (40) and/or a surgical table pedestal base (50).

11. Device according to one of the preceding Claims, **characterized by** the fact that the surgical table (30) has at least one position determination unit which records the positions of the components (32 through 46) in relation to one another and/or in space, whereby the position determination unit comprises at least one sensor.

12. Method for controlling a surgical table, having at least three components (32 through 46), the position of which can be modified with the assistance of operating elements (14 through 28), in which, with the assistance of at least two drive units (41), the position of the first component and the second component (44) relative to one another and relative to the third component (40) can be modified, the drive units (41) are controlled with the assistance of a control unit (52), at any rate at least one first position of the first component (42) and the second component (44) relative to one another and relative to the third component (40) is stored in the control unit (52), upon actuation of a first operating element (18) the at least two drive units (41) are controlled in such a way by the control unit, that the components (32 through 46) are moved into the stored position, at least one preset control parameter is stored in the control unit (52), and in which the at least two drive units (41) are controlled by the control unit (52) to move the components (32 through 46) at least from one of the second positions that differs from the first stored position into the first stored position, depending on the stored control parameter,
whereby the drive units (41) comprise at least one stepper motor and upon modification of the position of the components (32 through 46), the control unit (52) records and stores as information, the sequence of steps and/or the timing of the sequence of steps of the stepper motor, and/or,
whereby the control unit (52) upon execution of every positional modification of the components (32 through 46) records and stores as information, the adjustment travel, the timing of the adjustment travel, the adjustment travel velocity and/or the timing of the adjustment travel velocity of a linear drive serving as a drive unit (41), in particular hydraulic cylinders or screw drives, and
whereby the control unit (52) additionally or alternatively records and saves as information, the duration of the actuation of the respective drive unit (41) and/or velocity level actuated for this drive unit (41).

## Revendications

1. Dispositif de commande d'une table d'opération,
comprenant une table d'opération (30) avec
au moins trois composants (32 à 46) pouvant être modifiés dans leur position à l'aide d'éléments d'actionnement (14 à 28),
au moins deux unités d'entraînement (41) à l'aide desquelles la position du premier composant (42) et du deuxième composant (44) l'un par rapport à l'autre et par rapport au troisième composant (40) peut être modifiée,
une unité de commande (52) pour la commande des unités d'entraînement (41), dans lequel dans l'unité de commande (52), au moins une première position du premier composant (42) et du deuxième composant (44) l'un par rapport à l'autre et par rapport au troisième composant (40) est enregistrée,
dans lequel
l'unité de commande (52) commande, lors de l'activation d'un premier élément de commande (18), les unités d'entraînement (41) de telle sorte que les composants (32 à 46) sont déplacés dans la position enregistrée,
dans lequel dans l'unité de commande (52), au moins un paramètre de commande est enregistré par défaut, et dans lequel l'unité de commande (52) commande les au moins deux unités d'entraînement (41) pour le déplacement des composants (32 à 46) au moins d'une deuxième position différente de la première position enregistrée dans la première position enregistrée en fonction du paramètre de commande enregistré,
**caractérisé en ce que**
les unités d'entraînement (41) comprennent au moins un moteur pas-à-pas et l'unité de commande (52) lors de la modification de l'emplacement des composants (32 à 46) saisit la séquence de pas et/ou l'évolution temporelle de la séquence de pas du moteur pas-à-pas et l'enregistre en tant qu'information, et/ou
dans lequel l'unité de commande (52), lors de la réalisation de chaque modification d'emplacement des composants (32 à 46), saisit la course de déplacement, l'évolution temporelle de la course de déplacement, la vitesse de déplacement et/ou l'évolution temporelle de la vitesse de déplacement d'un entraînement linéaire servant d'unité d'entraînement (41), en particulier d'un cylindre hydraulique ou d'un entraînement à broche et les enregistre en tant qu'information, et
dans lequel l'unité de commande (52) saisit en outre ou alternativement la durée de l'activation de l'unité d'entraînement respective (41) et/ou la vitesse activée pour cette unité d'entraînement (41) et les enregistre en tant qu'information.

2. Dispositif selon la revendication 1, **caractérisé en ce que** le paramètre de commande comprend au moins une condition à suivre lors du mouvement des composants (32 à 46).

3. Dispositif selon la revendication 1 ou 2, **caractérisé en ce que** le paramètre de commande comprend au moins l'une des options suivantes :
des degrés de liberté autorisés de mouvement d'un composant (32 à 46),
des plages angulaires autorisées des angles d'emplacement des composants (32 à 46) les uns par rapport aux autres,
le mouvement parallèle des composants (32 à 46),
le mouvement sériel des composants (32 à 46),
des mouvements alternatifs des composants (32 à 46),
le mouvement séquentiel des composants (32 à 46),
des vitesses de déplacement autorisées des composants (32 à 46),
des évolutions de vitesse de déplacement autorisées de la vitesse de déplacement des composants (32 à 46),
des tendances autorisées des composants (32 à 46) dans l'espace, et/ou
des séquences de déroulement de mouvement enregistrées entre plusieurs positions des composants (32 à 46).

4. Dispositif selon l'une des revendications précédentes, **caractérisé en ce qu'**une position zéro de la table d'opération (30), une position de transfert pour le transfert d'une surface de support du patient (31) de la table d'opération (30) sur un chariot de transport, une position de sortie, spécifique de l'opération, de la table d'opération (30) et/ou une position d'intervention, spécifique de l'opération, de la table d'opération (30) est enregistrée en tant que position.

5. Dispositif selon l'une des revendications précédentes, **caractérisé en ce qu'**une première position et au moins une deuxième position sont enregistrées par défaut, dans lequel l'unité de commande (52) règle la première position et la deuxième position l'une après l'autre par la manoeuvre multiple du premier élément d'actionnement (18) ou dans lequel l'unité de commande (52) règle la première position lors de la manoeuvre du premier élément d'actionnement (18) et la deuxième position lors de la manoeuvre du deuxième élément d'actionnement (24) à l'aide des unités d'entraînement (41).

6. Dispositif selon l'une des revendications précédentes, **caractérisé en ce que** l'unité de commande (52) arrête le mouvement des composants (32 à 46) lors de la désactivation du premier et/ou du deuxième élément d'actionnement (18, 24) également lorsque la première position enregistrée et/ou la deuxième position des composants (32 à 46) n'est pas encore atteinte.

7. Dispositif selon l'une des revendications précédentes, **caractérisé en ce qu'**une position des composants (32 à 46) réglable de façon quelconque à l'aide d'autres ou des autres éléments d'actionnement (14 à 22) peut être enregistrée par un opérateur en tant que position par défaut.

8. Dispositif selon l'une des revendications précédentes, **caractérisé en ce que** dans l'unité de commande (52), une deuxième position du premier composant (42) et du deuxième composant (44) l'un par rapport à l'autre et par rapport au troisième composant (40), différente de la première position enregistrée, est enregistrée, **en ce qu'**un opérateur modifie à l'aide d'autres éléments d'actionnement (14 à 22), la position des composants (40, 42, 44) de la première position enregistrée à la deuxième position enregistrée, **en ce que** l'unité de commande (52) enregistre l'évolution du mouvement ainsi généré et/ou l'information de commande générée en outre par l'unité de commande (52) pour la commande des unités d'entraînement (41) et **en ce que** le déroulement du mouvement et/ou l'information de commande peut être consultée pour réaliser à nouveau le mouvement entre les deux positions à l'aide du premier élément d'actionnement (18) et/ou du deuxième élément d'actionnement (24) .

9. Dispositif selon la revendication 8, **caractérisé en ce que** lors d'un mouvement des composants (32 à 46) de la première position enregistrée à la deuxième position enregistrée, un déroulement de mouvement complètement inversé des composants (32 à 46) s'effectue comme lors du mouvement des composants (32 à 46) de la deuxième position enregistrée à la première position enregistrée.

10. Dispositif selon l'une des revendications précédentes, **caractérisé en ce que** le premier composant (42) et le deuxième composant (44) sont respectivement un composant d'une surface de support du patient (31) de la table d'opération (30), et **en ce que** le troisième composant est une colonne de table d'opération (40) et/ou un pied de colonne de table d'opération (50) .

11. Dispositif selon l'une des revendications précédentes, **caractérisé en ce que** la table d'opération (30) a au moins une unité de saisie de position qui saisit la position des composants (32 à 46) les uns par rapport aux autres et/ou dans l'espace, dans lequel l'unité de saisie de la position comprend au moins un capteur.

12. Procédé de commande d'une table d'opération qui a au moins trois composants (32 à 46) pouvant être modifiés dans leur emplacement à l'aide d'éléments d'actionnement (14 à 28), dans lequel, à l'aide d'au moins deux unités d'entraînement (41), la position du premier composant et du deuxième composant (44) l'un par rapport à l'autre et par rapport au troisième composant (40) peut être modifiée, les unités d'entraînement (41) peuvent être commandées à l'aide d'une unité de commande (52), au moins une première position d'au moins le premier composant (42) et du deuxième composant (44) l'un par rapport à l'autre et par rapport au troisième composant (40) est enregistrée dans l'unité de commande (52), les au moins deux unités d'entraînement (41) sont commandées lors de l'activation d'un premier élément d'actionnement (18) par l'unité de commande de telle sorte que les composants (32 à 46) sont déplacés dans la position enregistrée, dans l'unité de commande (52), au moins un paramètre de commande est enregistré par défaut, et dans lequel les au moins deux unités d'entraînement (41) sont commandées pour le déplacement des composants (32 à 46) au moins d'une deuxième position différente de la première position enregistrée à la première position enregistrée par l'unité de commande (52) en fonction du paramètre de commande enregistré,
dans lequel les unités d'entraînement (41) comprennent au moins un moteur pas-à-pas et l'unité de commande (52) lors de la modification de la position des composants (32 à 46) saisit la séquence de pas et/ou l'évolution temporelle de la séquence de pas du moteur pas-à-pas et l'enregistre en tant qu'information, et/ou
dans lequel l'unité de commande (52), lors de la réalisation de chaque modification d'emplacement des composants (32 à 46), saisit la course de déplacement, l'évolution temporelle de la course de déplacement, la vitesse de déplacement et/ou l'évolution temporelle de la vitesse de déplacement d'un entraînement linéaire servant d'unité d'entraînement (41), en particulier d'un cylindre hydraulique ou d'un entraînement à broche et les enregistre en tant qu'information, et
dans lequel l'unité de commande (52) saisit en outre ou alternativement la durée de l'activation de l'unité d'entraînement respective (41) et/ou la vitesse activée pour cette unité d'entraînement (41) et les enregistre en tant qu'information.
